# EUROPEAN PATENT SPECIFICATION

(11) **EP 2 992 106 B1**
(45) Date of publication and mention of the grant of the patent: **13.06.2018**
(21) Application number: 14722148.5
(22) Date of filing: 28.04.2014
(51) Int. Cl.: C12P 17/12

(54) **ENZYMATIC PROCESS FOR THE PRODUCTION OF (R)-3-QUINUCLIDINOL**
ENZYMATISCHES VERFAHREN ZUR HERSTELLUNG VON (R)-3-QUINUCLIDINOL
PROCÉDÉ ENZYMATIQUE POUR LA PRODUCTION DE (R)-3-QUINUCLIDINOL

(30) Priority: 30.04.2013 DE 102013104418
(43) Date of publication of application: 09.03.2016
(73) Proprietor: Cambrex IEP GmbH, 65203 Wiesbaden (DE)
(72) Inventor: TSCHENTSCHER, Anke, 65347 Eltville (DE); DUPONT, Maria, 50354 Hürth (DE); GUPTA, Antje, 65207 Wiesbaden (DE)
(74) Representative: Plate, Jürgen
(86) International application number: PCT/EP2014/058547
(87) International publication number: WO 2014/177492

(56) References cited:
- EP-A1- 2 423 320
- CN-A- 103 555 608
- JP-A- 2008 212 144
- HOU ET AL: "Expression, purification, crystallization and X-ray analysis of 3-quinuclidinone reductase from Agrobacterium tumefaciens", ACTA CRYSTALLOGRAPHICA SECTION F, vol. F68, 2012, pages 1237-1239, XP002726818, cited in the application
- ISOTANI ET AL: "Gene cloning and characterization of two NADH-dependent 3-quinuclidinone reductases from Microbacterium luteolum JCM 9174", APPLIED AND ENVIRONMENTAL MICROBIOLOGY, vol. 79, 21 December 2012 (2012-12-21), pages 1378-1384, XP002726819,
- TANOKURA ET AL: "Recent progress and development of crystal structure analysis of enzymes and other proteins", NIHON KESSHO GAKKAISHI, vol. 52 2010, pages 8-13, XP002726825, Retrieved from the Internet: URL:https://www.jstage.jst.go.jp/article/j crsj/52/1/52_1_8/_pdf [retrieved on 2014-07-08]
- WANG ET AL: "Microbial stereospecific reduction of 3-quinuclidinone with newly isolated Nocardia sp. and Rhodococcus erythropolis", JOURNAL OF MOLECULAR CATALYSIS B: ENZYMATIC, vol. 88, 24 November 2012 (2012-11-24), pages 14-19, XP002726826,
- HOU ET AL: "Structural basis for high substrate-binding affinity and enantioselectivity of 3-quinuclidinone reductase AtQR", BIOCHEMICAL AND BIOPHYSICAL RESEARCH COMMUNICATION, vol. 446, 15 March 2014 (2014-03-15), pages 911-915, XP028649471,
- ZHANG ET AL: "Highly efficient synthesis of (R)-3-quinuclidinol in a space-time yield of 916 g L(-1)d(-1) using a new bacterial reductase ArQR", ORGANIC LETTERS, vol. 15, 19 September 2013 (2013-09-19), pages 4917-4919, XP002726823,
- TAKESHITA ET AL: "Structural basis of stereospecific reduction by quinuclidinone reductase", AMB EXPRESS, vol. 4, 7 February 2014 (2014-02-07), pages 1-10, XP002726820,

### SEQUENCE LISTING

<110> Cambrex IEP GmbH
<120> Biocatalytic process for the production of (R)-3-Quinuclidinol
<130> MB
<160> 10
<170> PatentIn version 3.5
<210> 1
   <211> 256
   <212> PRT
   <213> Mycobacterium vanbaalenii
<400> 1
   Met Thr Arg Thr Val Val Val Thr Gly Ala Gly Ser Gly Ile Gly Arg
   1               5                   10                  15
   Ala Ile Ala Asn Thr Leu Ala Gln Arg Asn Trp Arg Val Val Val Thr
               20                  25                  30
   Asp Val Asn Ala Ala Ala Ala Ser Glu Val Ala Ala Ala Leu Pro Asn
           35                  40                  45
   Pro Pro Ala Gly His Glu Ser Ala Gln Leu Asp Val Thr Ser Pro Glu
       50                  55                  60
   Asn Ala Ala Ala Val Ala Ser Asp Val Ser Glu Arg Leu Gly Leu Asp
   65                  70                  75                  80
   Ala Trp Val Ser Asn Ala Gly Ile Ser Phe Met Arg Arg Phe Leu Asp
                   85                  90                  95
   Ala Pro Ile Glu Arg Tyr Glu Gln Thr Met Asp Val Asn Leu Lys Gly
               100                 105                 110
   Val Phe Val Cys Gly Gln Ala Ala Ala Arg Glu Met Val Arg Ala Gly
           115                 120                 125
   Ile Pro Gly Met Ile Val Asn Thr Ala Ser Met Ala Gly Lys Gln Gly
       130                 135                 140
   Arg Val Pro Phe Leu Ser Asp Tyr Val Ala Ser Lys Phe Gly Val Val
   145                 150                 155                 160
   Gly Leu Thr Gln Ala Met Ala Tyr Glu Leu Gly Glu His Gly Ile Thr
                   165                 170                 175
   Val Asn Cys Val Cys Pro Gly Phe Val Glu Thr Pro Met Gln Ser Arg
   180 185 190
   Glu Leu Gln Trp Glu Ala Glu Leu Arg Gly Thr Thr Pro Asp Gly Val
   195 200 205
   Arg Ala Met Met Ile Ala Asp Thr Pro Leu Gly Arg Leu Glu Gln Ala
   210 215 220
   Asp Asp Val Ala Arg Ala Val Ala Phe Leu Leu Ser Asp Asp Ala Arg
   225 230 235 240
   Phe Ile Thr Gly Glu Ala Leu Ala Val Asn Gly Gly Ala Tyr Met Asp
   245 250 255
<210> 2
   <211> 771
   <212> DNA
   <213> Mycobacterium vanbaalenii
<400> 2
   atgacgagaa ctgtggtcgt gaccggagcg ggatccggta tcggacgagc gatcgccaac 60
   acactggcgc aacgaaattg gcgggtggtg gtcaccgacg tcaatgccgc cgccgcaagc 120
   gaggtcgcag cggcactgcc gaatccgccc gcaggccatg aatccgcaca actggacgtc 180
   acctcccccg agaacgcggc cgccgtggca tccgacgtct cggagcggct cgggctcgac 240
   gcgtgggtga gcaacgccgg catctcgttc atgcgccgct tcctcgacgc ccccatcgag 300
   cgctacgagc agaccatgga cgtgaatctc aagggcgtgt tcgtgtgcgg gcaggccgcg 360
   gcgcgggaga tggtgcgcgc cggcattccc gggatgatcg tgaacaccgc ctcgatggcg 420
   ggcaagcagg gccgggtgcc gttcctgtcg gactacgtcg cctcgaaatt cggggtggtc 480
   gggttgaccc aggcgatggc ctacgaactc ggcgagcatg gcatcacagt caactgcgtg 540
   tgccctggct ttgtcgaaac ccccatgcag tcaagggaat tgcagtggga ggccgagctg 600
   cgtggcacca cccccgacgg tgtccgcgcc atgatgatcg ccgacacgcc gctgggtcgc 660
   cttgagcagg ccgacgacgt cgcccgcgcc gtggccttcc tgctctccga cgacgcccgt 720
   ttcatcaccg gcgaagcgct cgccgtcaac ggcggcgcct acatggactg a 771
<210> 3
   <211> 264
   <212> PRT
   <213> Mycobacterium smegmatis
<400> 3
   Met Ser Thr Pro Ala Asn Ser Thr Pro Asp Thr Pro Ser Thr Val Val
   1               5                   10                  15
   Val Thr Gly Ala Gly Ser Gly Ile Gly Arg Ala Ile Ala Thr Thr Leu
   20 25 30
   Ala Glu Arg Gly Trp Arg Val Val Ala Thr Asp Val Asp Val Ala Ala
   35 40 45
   Ala Asp Glu Thr Ala Ser Ser Leu Ser Gly Ser Gly His Glu Ser Ala
   50 55 60
   Arg Leu Asp Val Thr Asp Pro Ala Gln Ala Ser Ala Leu Ala Asp Asp
   65 70 75 80
   Val Ala Asp Arg Ile Gly Leu Gly Ala Trp Val Ser Asn Ala Gly Ile
   85 90 95
   Ser Ala Met Ala His Phe Val Asp Ile Ser Ala Glu Gln Leu Asp Arg
   100 105 110
   Ser Leu Asp Val Asn Leu Lys Gly Val Phe Phe Cys Gly Gln Ala Ala
   115 120 125
   Ala Arg Ala Met Ile Arg Thr Gly Val Arg Gly Thr Ile Val Asn Thr
   130 135 140
   Ala Ser Met Ala Ala Lys Gln Gly Arg Val Pro Phe Leu Ala Asp Tyr
   145 150 155 160
   Val Ala Ser Lys Phe Gly Val Leu Gly Leu Thr Gln Ala Met Ala Phe
   165 170 175
   Glu Leu Ala Ala His Gly Ile Thr Val Asn Ser Val Cys Pro Gly Phe
   180 185 190
   Val Ala Thr Pro Met Gln Thr Arg Glu Leu Glu Trp Glu Ala Lys Leu
   195 200 205
   Ser Gly Ser Thr Pro Asp Gly Val Arg Gln Ser Trp Ile Asp Ala Thr
   210 215 220
   Pro Leu Gly Arg Leu Gln Thr Pro Asp Asp Val Ala Arg Ala Val Ala
   225 230 235 240
   Phe Leu Val Ser Glu Asp Ala Arg Phe Ile Thr Gly Glu Ala Leu Ser
   245 250 255
   Val Asn Gly Gly Ala Tyr Met Asp
   260
<210> 4
   <211> 795
   <212> DNA
   <213> Mycobacterium smegmatis
<400> 4
   atgagcaccc ccgcgaacag cactcccgac acccccagca ccgtcgtcgt caccggcgcc      60
   ggatccggca tcggccgcgc gatcgccacc acgctcgccg aacgcggctg gcgtgtggtc     120
   gccaccgacg tcgacgtcgc cgcggccgac gagacggcct catccctgag cggcagcggc     180
   cacgagtcgg cgcgtctcga cgtcaccgat cccgcgcagg cgtccgcact ggccgacgac     240
   gtcgccgacc gcatcgggct gggggcctgg gtcagcaacg cgggcatctc cgcgatggcg     300
   cacttcgtcg acatctccgc cgaacaactc gaccgcagtc tcgacgtcaa cctcaagggc     360
   gtgttcttct gtggtcaggc cgcggcccgc gccatgatcc gcaccggtgt gcgcggcacg     420
   atcgtcaaca ccgcatcgat ggcggcaaag cagggccggg taccgttcct cgcggactat     480
   gtcgcgtcca agttcggcgt gctgggactc acccaggcga tggccttcga acttgccgca     540
   cacggcatca cggtcaacag tgtctgcccc ggtttcgtgg caacaccgat gcagacacga     600
   gaattggagt gggaggcgaa actcagcggc tcgacgcccg acggcgtacg ccagtcctgg     660
   atcgacgcga cgccgctggg ccgcctgcaa actcccgacg acgtcgcccg cgcggtggcg     720
   ttcctggtct ccgaagatgc ccgcttcatc accggagagg cgctgtcggt caacggcggt     780
   gcctacatgg actga                                                      795
<210> 5
   <211> 259
   <212> PRT
   <213> Caldilinea aerophila
<400> 5
   Met Arg Leu Arg Gly Lys Ile Ile Gly Ile Thr Gly Ala Gly Ser Gly
   1               5                   10                  15
   Ile Gly Lys Ala Ala Ala Leu Ala Leu Ala Arg Glu Gly Ala Ala Leu
               20                  25                  30
   Ala Val Thr Asp Phe Asn Leu Asp Trp Ala Gln Gln Thr Ala Ala Asp
           35                  40                  45
   Ile Arg Ser Gln Gly Gly Glu Ala Thr Ala Trp Arg Leu Asp Val Thr
       50                  55                  60
   Asp Gln Ala Glu Ala Glu Arg Val Leu Gln Ala Leu Ile Asp Arg Tyr
   65                  70                  75                  80
   Gly Arg Leu Asp Val Trp Val Asn Asn Ala Gly Val Ser Thr Met Asn
                   85                  90                  95
   Arg Phe Val Asp Leu Thr Glu Arg Asp Trp Asp Tyr Asn Met Asn Val
               100                 105                 110
   Asn Ala Lys Gly Val Phe Leu Cys Ser Gln Val Ala Ala Arg Arg Met
           115                 120                 125
   Ile Ala Gln Gly Gly Gly Lys Ile Ile Asn Val Ala Ser Met Ala Gly
       130                 135                 140
   Lys Arg Gly Asn Ala Pro Phe Leu Ala His Tyr Val Ala Ser Lys Phe
   145                 150                 155                 160
   Ala Val Ile Gly Leu Thr Gln Ala Met Ala Gly Glu Leu Ala Pro Tyr
                   165                 170                 175
   His Ile Thr Val Asn Ala Val Cys Pro Gly Tyr Val Arg Thr Ser Met
               180                 185                 190
   Gln Glu Arg Glu Val Glu Trp Glu Ala Met Leu Arg Gly Val Thr Pro
           195                 200                 205
   Glu Ala Val Arg Gln Leu Tyr Ile Gln Asp Thr Pro Leu Arg Arg Leu
       210                 215                 220
   Glu Thr Pro Glu Asp Val Ala Lys Val Ile Val Phe Leu Ala Ser Glu
   225                 230                 235                 240
   Asp Ala Asp Phe Ile Thr Gly Glu Ala Ile Asn Val Asn Gly Gly Ala
                   245                 250                 255
   Trp Met Asp
<210> 6
   <211> 780
   <212> DNA
   <213> Caldilinea aerophila
<400> 6
   atgcgactca ggggcaaaat tatcggcatc accggcgctg gttcaggcat tggcaaggcg    60
   gcggctctag cgctggcgcg ggagggcgcc gcattggccg tgacggactt caacctggat   120
   tgggcgcagc aaacagccgc agacatccgc agccagggcg gcgaggcgac agcatggcgg   180
   cttgacgtga ccgaccaggc cgaggcagag cgcgtgctgc aggcgctgat cgaccgttac   240
   ggccggctcg acgtctgggt gaacaacgca ggcgtctcga ccatgaaccg ctttgttgac   300
   ctcactgagc gggactggga ctataacatg aacgtcaacg ccaaaggcgt ttttctctgc    360
   agccaggtcg ccgcccgacg catgatcgcc caaggaggtg ggaagatcat caacgtcgcc   420
   tccatggcag gcaaacgagg caacgcacct ttcctggctc attatgttgc cagcaaattt   480
   gctgtaatcg ggctgacgca agcgatggcg ggcgaactgg cgccttatca catcaccgtc   540
   aacgccgttt gccctggcta tgtgcgcaca tctatgcaag agcgagaggt ggagtgggag   600
   gcaatgctgc gtggggttac acccgaagcg gtgcgacaac tgtatatcca ggatacacct   660
   ctgcgacgcc tcgaaacacc cgaagatgtg gcgaaagtca ttgtttttct tgcttcagag   720
   gacgcagact tcatcacggg cgaagcgatt aacgtcaacg gcggcgcgtg gatggattaa   780
<210> 7
   <211> 266
   <212> PRT
   <213> Starkea novella
<400> 7
   Met Ile Pro Ala Thr Ala Pro Thr Pro Val Tyr Pro Glu Leu Ser Gly
   1               5                   10                  15
   Arg Leu Ala Phe Val Thr Gly Ala Ala Thr Gly Ile Gly Arg Ala Ile
               20                  25                  30
   Ala Thr Ala Leu Ala Arg Gln Gly Val Arg Val Ala Ile Gly Asp Ile
           35                  40                  45
   Asn Leu Gly Ala Ala Glu Asp Ala Ala Ala Ala Ile Gly Gly Gly Ala
       50                  55                  60
   Val Ala Val Glu Val Asp Val Arg Arg Arg Ala Ser Val Glu Ala Ala
   65                  70                  75                  80
   Phe Ala Arg Val Leu Glu Leu Leu Gly Gly Cys Asp Leu Leu Val Ala
                   85                  90                  95
   Asn Ala Gly Val Ser Thr Met Gln Ala Ala Leu Glu Ile Thr Asp Gln
               100                 105                 110
   Glu Trp Asp Phe Asn Phe Asp Val Asn Thr Arg Gly Val Phe Leu Thr
           115                 120                 125
   Asn Gln Ile Val Ala Arg His Phe Val Ala Thr Gly Lys Gly Cys Ile
       130                 135                 140
   Val Asn Thr Ala Ser Leu Ala Ala Lys Val Gly Ala Pro Leu Leu Ala
   145                 150                 155                 160
   His Tyr Ser Ala Ser Lys Phe Ala Val Leu Gly Trp Thr Gln Ala Leu
                   165                 170                 175
   Ala Arg Glu Leu Ala Pro Lys Gly Ile Arg Val Asn Ala Val Cys Pro
               180                 185                 190
   Gly Phe Val Ala Thr Gly Met Gln Ser Arg Glu Val Gln Trp Glu Ala
           195                 200                 205
   Thr Leu Arg Gly Val Thr Pro Gln Arg Val Ile Asp Asp Tyr Ile Ala
       210                 215                 220
   Gln Thr Pro Leu Gly Arg Leu Glu Gln Pro Glu Asp Val Ala Asp Val
   225                 230                 235                 240
   Val Val Phe Leu Cys Ser Glu Gln Ala Arg Phe Met Thr Gly Gln Gly
                   245                 250                 255
   Val Asn Val Thr Gly Gly Val Tyr Thr Thr
               260                 265
<210> 8
   <211> 801
   <212> DNA
   <213> Starkea novella
<400> 8
   atgatccccg cgaccgcccc gacgcccgtc taccccgaac tttccggccg gctggccttc 60
   gtgacggggg cggcgacggg catcgggcgt gccatcgcca cggctctggc gcgccagggc 120
   gtgcgggtcg cgatcggcga catcaatctc ggcgcggccg aggacgcggc ggcggcgatc 180
   ggcggcggtg ccgtggcggt cgaggtggat gtgcgccggc gcgcctccgt cgaggcggcg 240
   ttcgcccgcg tgctggagtt gctcggcggc tgcgacctgc tggtcgccaa tgccggcgtc 300
   tcgaccatgc aggcggcgct cgagatcacc gaccaggagt gggacttcaa cttcgacgtc 360
   aacacccgcg gcgtgttcct caccaaccag atcgtcgccc ggcatttcgt ggcgaccggc 420
   aagggctgca tcgtcaacac cgcctcgctc gccgccaagg tcggggcgcc gctgctggcg 480
   cattactcgg cctccaaatt cgccgtcctc ggctggacgc aggcgctggc ccgcgagctc 540
   gcccccaagg gcatccgcgt caatgccgtc tgcccgggct tcgtggcgac cggcatgcag 600
   tcgcgcgagg tgcagtggga ggcgacgctg cgcggcgtga ccccgcagcg cgtgatcgac 660
   gactacatcg cccagacccc gctcggccgg ctggagcagc cggaggacgt cgccgacgtg 720
   gtggtgttcc tgtgctcgga gcaggcccgc ttcatgaccg ggcagggcgt caacgtcacc 780
   ggcggcgtct acacgacctg a 801
<210> 9
   <211> 264
   <212> PRT
   <213> Oceanithermus profundus
<400> 9
   Met Leu Glu Lys Arg Thr Ala Leu Ile Thr Gly Ala Gly Gly Gly Ile
   1 5 10 15
   Gly Ala Ala Val Ala His Arg Leu Ala Arg Glu Gly Ala Lys Val Trp
   20 25 30
   Val Thr Asp Arg Asp Leu Asp Ala Ala Glu Ala Thr Ala Ala Ala Ile
   35 40 45
   Arg Glu Ala Gly Gly Arg Ala Arg Ala Arg Arg Val Asp Val Thr Arg
   50 55 60
   Arg Glu Glu Leu Glu Ala Ala Cys Ala Ala Ala Tyr Ala Glu Asp Gly
   65 70 75 80
   Arg Val Asp Leu Val Val Ala Asn Ala Gly Val Ser Thr Met Arg Pro
   85 90 95
   Phe Leu Glu Leu Thr Asp Glu Asp Trp Ala Phe Asn Phe Asp Val Asn
   100 105 110
   Ala Arg Gly Thr Phe Tyr Thr Leu Gln Thr Phe Ala Arg Arg Met Lys
   115 120 125
   Asp Gln Ala Pro Met Pro Gly Ser Ser Leu Arg Gly Lys Leu Ile Ala
   130 135 140
   Val Ala Ser Met Ala Ala Arg Gln Ala Ala Pro Trp Leu Ala His Tyr
   145 150 155 160
   Ser Ala Ser Lys Phe Ala Val Leu Gly Leu Val Gln Ala Ala Ala Lys
   165 170 175
   Glu Leu Ala Pro Phe Arg Ile Thr Val Asn Ala Val Asn Pro Gly Phe
   180 185 190
   Val Lys Thr Ser Met Gln Glu Arg Glu Ile Ala Trp Glu Ala Arg Leu
   195 200 205
   Arg Gly Thr Thr Pro Glu Ala Val Val Ala Asp Tyr Leu Ala Gln Thr
   210 215 220
   Pro Leu Gly Arg Leu Glu Arg Pro Glu Asp Val Ala Gly Val Val Ala
   225 230 235 240
   Phe Leu Ala Gly Pro Asp Ala Asp Phe Ile Thr Gly Glu Ala Val Glu
   245 250 255
   Val Asn Gly Gly Ala Trp Ile Phe
   260
<210> 10
   <211> 795
   <212> DNA
   <213> Oceanithermus profundus
<400> 10
   atgcttgaga aaagaacggc actgatcacc ggcgccggcg gcggcatcgg cgccgccgtg    60
   gcccaccggc tcgcgcgcga aggcgccaag gtctgggtca cggaccgcga cctcgacgcc   120
   gcggaggcga cggccgcggc catccgggag gcgggcgggc gcgcccgcgc gcggcgcgtg   180
   gacgtcaccc gccgggaaga gctcgaggcc gcctgcgcag cggcctacgc cgaggacggg   240
   cgcgtcgacc tggtggtggc gaacgccggc gtgagcacga tgcgcccctt cctggagctg   300
   accgacgagg actgggcgtt caacttcgac gtcaacgccc gcgggacctt ctacaccctg   360
   cagaccttcg cccggcgcat gaaggaccag gcgccgatgc cgggaagctc gctgcggggg   420
   aagctgatcg ccgtggccag catggcggcg cgccaggcgg ccccctggct ggcccactac   480
   tccgcctcga agttcgcggt gctggggctg gtgcaggcgg cggcgaagga gctcgccccc   540
   ttccggatca ccgtcaacgc ggtgaacccg ggcttcgtca agacctcgat gcaggaacgc   600
   gaaatcgcct gggaggcgcg gctgcggggc accacccccg aggcggtcgt cgccgactac   660
   ctggcccaga ccccgctggg acggctggaa aggccggagg acgtcgccgg cgtggtggct   720
   ttcctggccg gccccgacgc cgacttcatc accggcgagg ccgtggaggt gaacggcggg   780
   gcctggatct tctga                                                    795

## Description

### Technical Field

The present invention relates to a process for the production of (R)-3-Quinuclidinol ((3R)-1-azabicyclo[2.2.2]octan-3-ol, CAS #: 25333-42-0) or a salt thereof (CAS #: 42437-96-7) of high optical purity, \ 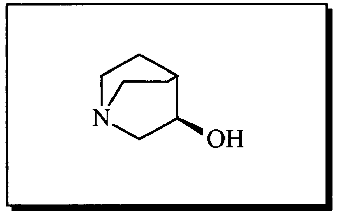 (R)-3-Quinuclidinol [(3R)-1-azabicyclo[2.2.2]octan-3-ol]
comprising reacting Quinuclidin-3-one with new Oxidoreductases by using preferably NADH as cofactor.

### Background art

(R)-3-Quinuclidinol is a valuable intermediate for the synthesis of a broad range of pharmaceuticals. In the pharmaceutical industry it is used for example as chiral synthon for cognition enhancer *Talsaclidine,* urinary incontinence agent *Solifenacin* or M₃ antagonist *Revatropate* for the treatment of asthma. There are different methods known for obtaining optically active Quinuclidinol such as a chemical reduction reaction using a metal catalyst (cf. JPH9-194480A), resolution of racemic mixtures of (±)-3-quinuclidinol esters by enzymatic hydrolysis reaction (cf. US5215918B, JPH10-136995A, JPH10-210997A, JPH9-194480A), and enzymatic reduction of Quinuclidin-3-one using whole cell biocatalysts or isolated enzymes (cf. JP10243795, JP11196890, JP2002153293, JP2000245495).

Preparation of optically pure (R)-3-Quinuclidinol through reduction with metal catalysts is reported to provide inadequate efficacy for industrial application, since the optical purity of the reduction product is low and additional purification steps are required for obtaining pure enantiomers.

The preparation of optically pure (R)-3-Quinuclidinol through resolution of racemic mixtures of (±)-3-quinuclidinol esters with protease is also not suited for industrial application, because of the necessity to remove remaining esters from the reaction mixture and the therewith associated high cost.

Preparation of optically pure (R)-3-Quinuclidinol through enzymatic reduction of Quinuclidin-3-one via whole biotransformation has been reported for cells of the genera *Nakazaewaea, Candida, Proteus, Rhodotorula, Sporidiolobus, Rhodosporidium, Schizosaccharomyces, Cryptococcus, Trichisporon, Gordonia, Nocardia, Alcaligenes, Corynebacterium, Arthrobacter, Filobasisdium, Aureobasidium, Yarrowia, Geotrichum, Tsukamurella, Kurthia, Microbacterium, Kluyveromyces, Acremonium and Mucor* (cf. JP10243795, JP11196890, JP2002153293, JP2000245495). The impurities incurred from microbial cells and or side reactions caused by other enzymes contained in the microorganisms lowers the optical purity and product yield.

Preparation of(R)-3- Quinuclidinol through oxidoreduction processes using isolated enzymes is also known in the art and has been reported for enzymes from plants of the genera *Datura* and *Hyoscyamus* (Patent JP2003230398), as well as enzymes from *Rhodotorula* (JP2007124922), *Burkholderia* (WO2010123062) and *Microbacterium luteolum* (Int J Mol Sci. 2012 Oct 19;13(10):13542-53). Recently an alcohol dehydrogenase, which reduces Quinuclidin-3-one has been crystallized from Agrobacterium tumerfacience (Acta crystallografica 10(2012) 68 (Pt 10): 1237-1239).
Because of plant origin the enzymes derived from *Datura* and *Hyoscyamus* are difficult to manufacture industrially. The enzymes originating from *Rhodotorula* and *Burkholderia* require expensive NADPH as cofactor and Glucose Dehydrogenase for NADPH regeneration, thus rendering the manufacturing processvery costly.

To date, the most efficient process for the production of (R)-3-Quinuclidinol is reported by Isotani et al. (Int J Mol Sci. 2012 Oct 19;13(10):13542-53) for the reduction process using enzymes from Microbacterium luteolum. Enzymes derived from *Microbacterium luteolum* require NADH as cofactor and a secondary alcohol dehydrogenase for cofactor regeneration in conjunction with 2-propanol as cosubstrate. However, in this enzymatic reduction process the substrate load is limited, supposedly due to the inhibitory effect of Quinuclidin-3-one on enzymatic activity. A maximal substrate load of 10 % (w/v) could be achieved by continuously adding substrate to the reaction batch, thus keeping the concentration of Quinuclidin-3-one in the reaction vessel and the concomitant enzyme inhibition at an adequate level. A conversion yield of 100 % at 15 % (w/v) substrate load was achieved using immobilized enzymes. However, immobilization of enzymes is elaborate and costly and imposes limitations on the reaction volume, that are not commensurate with the requirements of an industrial manufacturing process.

The article *"*Expression, purification, crystallization and X-ray analysis of 3-quinuclidinone reductase from Agrobacterium temefaciens"; F. Hou, T. Miyakawa, D. Takeshita, M. Kataoka, A. Uzura, K. Nagata, S. Shimizu, M. Tanokura; Acta Crystallographica Section F; 2012; pp. 1237-1239 describes overexpression, purification, crystallization and structure analysis of reductase AtQR from Agrobacterium tumefaciens, which catalyzes reduction of 3-quinuclidinone to (R)-3-quinuclidinol with NADH as cofactor. Recombinant AtQR was obtained by overexpression in Eschericia coli, purified, crystallized with NADH by sitting-drop vapor diffusion and subsequently analyzed by X-ray diffraction using synchrotron radiation at Photon Factory, Tsukuba.

The scientific paper "Structural basis for high substrate-binding affinity and enantioselectivity of 3-quinoclidinone reductase AtQR"; F. Hou, T. Miyakawa, M. Kataoka, D. Takeshita, S. Kumashiro, A. Uzura, N. Urano, K. Nagata, S. Shimizu, M. Tanokura; Biochem Biophys Res Comm; 2014; pp. 911-913 discloses the structure of AtQR from Agrobacterium tumefaciens, which catalyzes reduction of 3-quinuclidinone to (R)-3-quinuclidinol with NADH as cofactor. Based on the structure of AtQR the authors calculate substrate binding affinity and explain the catalytic mechanism.

JP 2008-212144 pertains to an alcohol dehydrogenase that is capable of asymmetrically reducing 3-quinuclidinone or a salt thereof to (R)-3-quinuclidinol using NADH as coenzyme at high optical purity and yield.

CN 103555608 A discloses a quininone reductase and its application for asymmetric synthesis of (R)-3-quinuclidinol.

The article *"*Highly Efficient Synthesis of (R)-3-Quinuclidinol in a Space-Time Yield of 916 g L-1 d-1 Using a New Bacterial Reductase ArQ "; W.-X. Zhang, G.-C Xu, L. Huang, J. Pan, H.-L. Yu, J.-H. Xu; Organic Letters; 2013 Vol. 15, 19; pp. 4917-4919 describes a keto reductase derived from *Agrobacterium radiobacter ECU2556* for enantioselective reduction of 3-quinuclidinone to (R)-3-quinuclidinol using NADH as coenzyme.

The scientific paper *"*Structural basis of stereospecific reduction by quinoclidinone reductase"; D. Takeshita, M. Kataoka, T. Miyakawa, K.-l. Miyazono, S. Kumashiro, T. Nagai, N. Urano, A. Uzura, K. Nagata, S. Shimizu, M. Tanokura; AMB Express; 2014 Vol. 4, 6; pp. 1-10 pertains to the structure and binding site of an NADPH-dependent 3-quinuclidinone reductase (RrQR) from Rodotorula rubra.

The number of known enzymes for reduction Quinuclidin-3-one is limited. Furthermore, these enzymes are not commercially available and the corresponding reduction processes have major drawbacks. Thus, there is a need for an efficient and industrially applicable method for producing (R)-3-Quinuclidinol with novel recombinant oxidoreductases used as biocatalyst in the reduction reaction.

An oxidoreductase suitable for an industrial reduction process for 3-Quinuclidinone must satisfy the following requirements:
1) commensurate expression in recombinant strain (for example in Escherichia coli);
2) high stability in organic solvents, particularly in water miscible organic solvents such as 2-propanol and 2-butanol;
3) enzymatic activity at high concentration of the substrate 3-Quinuclidinone without being adversely affected by substrate or product inhibition;
4) compliance with enzyme-coupled cofactor regeneration, in particular cofactor regeneration through alcohol dehydrogenase using secondary alcohols as cosubstrates.

The present invention has the object to provide a method for preparing (R)-3-Quinuclidinol by reducing Quinuclidin-3-one or a salt thereof using a cofactor and an isolated oxidoreductase, a recombinant organism expressing said oxidoreductase, or a preparation of an organism expressing said oxidoreductase, such as permeabilized, lysed or lyophilized cells thereof. The oxidoreductase is selected from
a) polypeptides with amino acid sequence SEQ ID NO:1 or SEQ ID NO:3 or SEQ ID NO: 5 or SEQ ID NO:7 or SEQ ID NO:9 of the sequence listing; or
b) polypeptides with an amino acid sequence derived from SEQ ID NO: 1 or SEQ ID NO:3 or SEQ ID NO: 5 or SEQ ID NO:7 or SEQ ID NO:9 through substitution, deletion or addition of one, two, three, four, five, six, seven, eight, nine, ten, eleven, twelve, thirteen, fourteen, fifteen, sixteen, seventeen, eighteen, nineteen, twenty, twentyone, twentytwo, twentythree, twentyfour, twentyfive, twentysix, twentyseven, twentyeight, twentynine, thirty, thirtyone, thirtytwo, thirtythree, thirtyfour, thirtyfive, thirtysix, thirtyseven, thirtyeight, thirtynine, or forty amino acid residues and which are capable of reducing Quinuclidin-3-one in conjunction with a cofactor; or
c) polypeptides with an amino acid sequence, wherein at least 65 % of the amino acids are identical to the amino acids of SEQ ID NO:1 or SEQ ID NO:3, or wherein at least 70 % of the amino acids are identical to the amino acids of SEQ ID NO:1 or SEQ ID NO:3, or wherein at least 75 % of the amino acids are identical to the amino acids of SEQ ID NO:1 or SEQ ID NO:3 or SEQ ID NO: 5 or SEQ ID NO:9, or wherein at least 85 % of the amino acids are identical to the amino acids of SEQ ID NO:1 or SEQ ID NO:3 or SEQ ID NO: 5 or SEQ ID NO:9, most preferably wherein at least 90 % of the amino acids are identical to the amino acids of SEQ ID NO:1 or SEQ ID NO:3 or SEQ ID NO: 5 or SEQ ID NO:9, and which are capable of reducing Quinuclidin-3-one in conjunction with a cofactor; or
d) polypeptides with an amino acid sequence, wherein at least 85 %, most preferably at least 90 % of amino acids are identical to the amino acids of SEQ ID NO:7, and which are capable of reducing Quinuclidin-3-one in conjunction with a cofactor; or
e) polypeptides that are encoded by a polynucleotide with nucleotide sequence SEQ ID NO: 2 or SEQ ID NO:4 or SEQ ID NO:6 or SEQ ID NO:8 or SEQ ID NO:10 of the sequence listing; or
f) polypeptides that are encoded by a polynucleotide with a nucleotide sequence which hybridizes to the full length complement of nucleotide sequence SEQ ID NO: 2 or SEQ ID NO:4 or SEQ ID NO:6 or SEQ ID NO:8 or SEQ ID NO:10 under stringent conditions, and which are capable of reducing Quinuclidin-3-one in conjunction with a cofactor; wherein the polypeptides comprise an amino acid sequence motif MQX₁REX₂X₃WEA, wherein each of X₁, X₂, X₃ are any of amino acid residues A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) or V (Val).

Preferably, the oxidoreductase is used in conjunction with cofactor NADH or NADPH.

It has been found that the polypeptides according to a) or b) or c) or d) or e) or f) are capable of efficiently reducing Quinuclidin-3-one to its corresponding (R)-alcohol. Surprisingly the sequence homology (identity) between the polypeptides for use in the present invention (see table 1) varies from 48 % to 72 %. However, the polypeptides have a specific amino acid sequence motif MQX₁REX₂X₃WEA in common, which is characteristic for the enzymatic activity of these proteins in the reduction of Quinuclidin-3-one.

| | SEQ ID NO: 1 Mycobacterium Vanbaalenii | SEQ ID NO:3 Mycobacterium smegmatis | SEQ ID NO:5 Caldilinea aerophila | SEQ ID NO: 7 Starkeya novella | SEQ ID NO: 9 Oceanithermus profundus |
|---|---|---|---|---|---|
| SEQ ID NO: 1 Mycobacteri um Vanbaalenii | 100 | 72% | 53% | 50% | 47% |
| SEQ ID NO: 3 Mycobacterium smegmatis | 72% | 100 | 53% | 48% | 45% |
| SEQ ID NO:5 Caldilinea aerophila | 53% | 53% | 100 | 50% | 57% |
| SEQ ID NO: 7 Starkeya novella | 50% | 48% | 50% | 100 | 52% |
| SEQ ID NO: 9 Oceanithermus profundus | 47% | 45% | 57% | 52% | 100 |

The sequence motif MQX₁ReX₂X₃WEA is located proximal to the C-terminus of the proteins. This finding is commensurate with the crystal structure and thereon based homology modeling of known short-chain alcohol dehydrogenases, which reveals that this motif forms a tail of a substrate binding pocket of the dehydrogenase, wherein amino residues M, Q, R, E, W, E, and A interact with the substrate. Thus it is postulated that short-chain alcohol dehydrogenases comprising said motif MQX₁REX₂X₃WEA are suited for reduction of Quinuclidin-3-one.

An oxidoreductase suitable for reduction of Quinuclidin-3-one, preferably to the corresponding R-alcohol, is understood to be a polypeptide which under optimized reaction conditions yields an enantiomeric excess of the R-alcohol of at least 50 %. Herein, optimized reaction conditions are understood to be reaction conditions under which a polypeptide yields maximum enantiomeric excess of the R-alcohol.
It has been found that the polypeptides for use in the present invention provide adequate oxidoreductase activities and can be used for reducing Quinuclidin-3-one preferably to (R)-3-Quinuclidinol. The achievable enantiomeric excess of the R-alcohol amounts to greater than or equal to 50%, preferably greater than or equal to 80% and particularly preferably greater than or equal to 95%. When using SEQ ID NO: 1 an enantiomeric excess of greater than or equal to 99% can be achieved.

Oxidoreductases capable of reducing the Quinuclidin-3-one to the corresponding a (R)-3-Quinuclidinol can be isolated from bacteria of the genus Mycobacterium, in particular from *Mycobacterium vanbaalenii* or from *Mycobacterium smegmatis,* from bacteria of the genus *Caldilinea,* in particular from *Caldilinea aerophila,* from bacteria of the genus *Starkeya,* in particular from *Starkeya novella,* from bacteria of the genus Oceanithermus, in particular from *Oceanithermus profundum.*

The polynucleotides coding for the oxidoreductase for use in the present invention are obtainable for example from the genomic DNA of the Bacterium Mycobacterium vanbaalenii strain PYR-1 or from *Mycobaterium smegmatis strain* ATCC 700084, from the bacteria of the genus *Caldilinea,* in particular from *Caldilinea aerophila* strain DSM 14535, from bacteria of the genus *Starkeya,* in particular from the *Starkeya novella* strain DSM 506, from bacteria of the genus *Oceanithermus,* in particular from *Oceanithermus profundum* by using known molecular biological techniques.

The organism producing the oxidoreductase is preferably a recombinant organism which overexpresses the oxidoreductase. Preferably, but not limited to, such recombinant organism is Escherichia coli. The oxidoreductase expressed by a recombinant organism can be used either in a completely purified state, in a partially purified state or as cells containing the polypeptide. The cells expressing the polypeptide can be used in a native, permeabilized, lysed or lyophilized state.

Further disclosed is the organism producing this oxidoreductase or a preparation of an organism expressing said oxidoreductase, such as permeabilized, lysed or lyophilized cells thereof, capable of enantioselectively reducing Quinuclidin-3-one to it's corresponding alcohol using a cofactor.

Preferably, the oxidoreductase is used in conjunction with cofactor NADH or NADPH.

In the present disclosure the term "amino acid sequence (A), wherein (P)% percent of amino acids are identical to the amino acids of amino acid sequence SEQ ID NO:(N)" refers to amino acid sequence (A) and SEQ ID NO:(N) being aligned over their full length and wherein either of aligned amino acid sequence (A) and aligned SEQ ID NO:(N) may comprise gap insertions relative to their respective non-aligned sequence. The percentage of identity is calculated by determining the number of positions at which identical amino acid residues occur in both aligned amino acid sequence (A) and aligned SEQ ID NO:(N), and dividing the number of matched positions by the total number of residues in the reference sequence. The result, multiplied by 100 yields the percentage of identity. The alignment of the sequences can be conducted by using the BLAST and BLAST 2.0 algorithms, which are described in Altschul et al., 1977, Nucleic Acids Res. 3389-3402 and Altschul et al., 1990, J. Mol. Biol. 215: 403-410.

The term "deleted amino acid" or "deletion" refers to modification of a polypeptide by removal of one or more amino acids, in both an internal and / or a terminal part of SEQ ID NO:1 or SEQ ID NO:3 or SEQ ID NO:5 or SEQ ID NO:7 or SEQ ID NO:9 while retaining the enzymatic activity.

The term "insertion" refers to modification of a polypeptide by addition of one or more amino acids, to the polypeptide at various positions in either an internal and/or terminal part of SEQ ID NO:1 or SEQ ID NO:3 or SEQ ID NO:5 or SEQ ID NO:7 or SEQ ID NO:9.

Substitution refers to replacement of one or more amino acids in SEQ ID NO:1 or SEQ ID NO:3 or SEQ ID NO:5 or SEQ ID NO:7 or SEQ ID NO:9 and can be conservative and / or non-conservative. Conservative amino acid substitution refers to the replacement of an amino acid residue with an amino acid residue having similar side chains or belonging to the same class of amino acids, e.g. from hydrophilic to hydrophilic, from hydrophobic to hydrophobic, from non-polar to non-polar, polar to polar, acidic to acidic, basic to basic, aromatic to aromatic. Examples of conservative substitutions are recited in the following table:

| **Residue** | **Conservative substitutions** |
|---|---|
| Ala, Val, Leu, Ile, Ser, Gly | Ala, Val, Leu, Ile, Ser, Gly |
| Asp, Glu | Asp, Glu |
| Gly, Met | Ala, Gly, Met |
| Lys, Arg | Lys, Arg |
| Asn, Gln, His | Asn, Gln, His |
| Trp, Phe, Tyr | Trp, Phe, Tyr |

Non-conservative substitution refers to replacement of an amino acid in the polypeptide with an amino acid with significantly different side chain and / or physicochemical properties. Non-conservative amino acid substitution may affect the structure of the polypeptide, its hydrophobicity and/or its net charge.

Accordingly, the disclosure also pertains to polypeptides with the hereafter recited amino acid sequence, comprising at each of positions 1-282 one amino acid selected from the corresponding selection group [...] enclosed in square brackets, wherein "-" designates a gap:

| Position | Amino acid |
|---|---|
| 1 | [-M] |
| 2 | [-FKLMRST] |
| 3 | [-L] |
| 4 | [-FM] |
| 5 | [-IPY] |
| 6 | [-AEP] |
| 7 | [-ADGQV] |
| 8 | [-DGQST] |
| 9 | [-ALMTV] |
| 10 | [-AGPQT] |
| 11 | [-AEHPST] |
| 12 | [-KNPQ] |
| 13 | [-IKQRSV] |
| 14 | [-FGHLTY] |
| 15 | [-APT] |
| 16 | [-DEGN] |
| 17 | [-LST] |
| 18 | [-AEKPRS] |
| 19 | [-DEGKST] |
| 20 | [-L] |
| 21 | [-KLPQR] |
| 22 | [-IKLNRSTV] |
| 23 | [-AIV] |
| 24 | [AFGILMV] |
| 25 | [ILV] |
| 26 | [T] |
| 27 | [G] |
| 28 | [AG] |
| 29 | [AG] |
| 30 | [AGRST] |
| 31 | [G] |
| 32 | [I] |
| 33 | [G] |
| 34 | [AKLRW] |
| 35 | [AG] |
| 36 | [ACIMV] |
| 37 | [AC] |
| 38 | [ADEHKLNQRST] |
| 39 | [ARST] |
| 40 | [LMY] |
| 41 | [AHSTV] |
| 42 | [AEGHKLQR] |
| 43 | [DEHQR] |
| 44 | [ADEGN] |
| 45 | [AFIRVW] |
| 46 | [AGKQRTW] |
| 47 | [ILV] |
| 48 | [AITVW] |
| 49 | [AIV] |
| 50 | [ACGIT] |
| 51 | [-D] |
| 52 | [-FILRV] |
| 53 | [-DN] |
| 54 | [-AEFGIKLPV] |
| 55 | [ADEGKRT] |
| 56 | [AFGLSW] |
| 57 | [AK] |
| 58 | [ADEKNQRS] |
| 59 | [ADEKNQRST] |
| 60 | [ATVW] |
| 61 | [ASV] |
| 62 | [ADEGHQS] |
| 63 | [ADEGS] |
| 64 | [ILV] |
| 65 | [-EGPRS] |
| 66 | [-ADEGKNQS] |
| 67 | [-A] |
| 68 | [-AEPQST] |
| 69 | [-DGLPST] |
| 70 | [-AEGRST] |
| 71 | [DEGHIMQR] |
| 72 | [-AHPST] |
| 73 | [-AEHILRTV] |
| 74 | [AGHLPS] |
| 75 | [-AHILRVW] |
| 76 | [AEGKPQRT] |
| 77 | [AILMV] |
| 78 | [D] |
| 79 | [V] |
| 80 | [RT] |
| 81 | [DEKNRS] |
| 82 | [APQR] |
| 83 | [ADENST] |
| 84 | [ADEGNQS] |
| 85 | [AILV] |
| 86 | [AEKQS] |
| 87 | [AKQRST] |
| 88 | [ALTV] |
| 89 | [ACFILRT] |
| 90 | [ADEQRS] |
| 91 | [ADEQRSTV] |
| 92 | [AILV] |
| 93 | [AILSTY] |
| 94 | [ADEG] |
| 95 | [AEIKLR] |
| 96 | [DFILMRY] |
| 97 | [GP] |
| 98 | [-GR] |
| 99 | [CILVY] |
| 100 | [DEGH] |
| 101 | [AILV] |
| 102 | [CLMVW] |
| 103 | [CHIV] |
| 104 | [ANS] |
| 105 | [N] |
| 106 | [A] |
| 107 | [G] |
| 108 | [IV] |
| 109 | [S] |
| 110 | [AFST] |
| 111 | [M] |
| 112 | [AEHNQR] |
| 113 | [AHKPR] |
| 114 | [AF] |
| 115 | [ILV] |
| 116 | [DE] |
| 117 | [AILV] |
| 118 | [PST] |
| 119 | [ADEIPV] |
| 120 | [DEGKQR] |
| 121 | [DEQR] |
| 122 | [FLWY] |
| 123 | [ADENQ] |
| 124 | [FQRY] |
| 125 | [NST] |
| 126 | [FLMV] |
| 127 | [ADNQS] |
| 128 | [IV] |
| 129 | [N] |
| 130 | [AILT] |
| 131 | [KR] |
| 132 | [AG] |
| 133 | [ITV] |
| 134 | [F] |
| 135 | [FILVY] |
| 136 | [ACSTV] |
| 137 | [GLNS] |
| 138 | [Q] |
| 139 | [AEIQTV] |
| 140 | [AFV] |
| 141 | [AGLT] |
| 142 | [R] |
| 143 | [AEHQR] |
| 144 | [FM] |
| 145 | [IKLSTV] |
| 146 | [ADEKNRS] |
| 147 | [AEQST] |
| 148 | [AEGNPQ] |
| 149 | [-IKPQRTV] |
| 150 | [-ADGKMPQRV] |
| 151 | [-P] |
| 152 | [-GNS] |
| 153 | [-ERS] |
| 154 | [-GS] |
| 155 | [-LV] |
| 156 | [-GR] |
| 157 | [-G] |
| 158 | [CKMSTV] |
| 159 | [IL] |
| 160 | [IV] |
| 161 | [AN] |
| 162 | [ITV] |
| 163 | [A] |
| 164 | [S] |
| 165 | [LM] |
| 166 | [A] |
| 167 | [AG] |
| 168 | [KR] |
| 169 | [IQRV] |
| 170 | [AG] |
| 171 | [-NR] |
| 172 | [AV] |
| 173 | [P] |
| 174 | [FLW] |
| 175 | [L] |
| 176 | [AST] |
| 177 | [DH] |
| 178 | [Y] |
| 179 | [SV] |
| 180 | [A] |
| 181 | [S] |
| 182 | [K] |
| 183 | [F] |
| 184 | [AG] |
| 185 | [V] |
| 186 | [ILV] |
| 187 | [G] |
| 188 | [LW] |
| 189 | [TV] |
| 190 | [Q] |
| 191 | [AG] |
| 192 | [AFLM] |
| 193 | [A] |
| 194 | [CFGKRY] |
| 195 | [E] |
| 196 | [FL] |
| 197 | [AG] |
| 198 | [ADEPSV] |
| 199 | [DFHKQRSY] |
| 200 | [GHKQRS] |
| 201 | [I] |
| 202 | [LRT] |
| 203 | [V] |
| 204 | [N] |
| 205 | [ACS] |
| 206 | [IV] |
| 207 | [CN] |
| 208 | [P] |
| 209 | [G] |
| 210 | [FY] |
| 211 | [V] |
| 212 | [AEKR] |
| 213 | [T] |
| 214 | [GPS] |
| 215 | [M] |
| 216 | [Q] |
| 217 | [EST] |
| 218 | [R] |
| 219 | [E] |
| 220 | [AILV] |
| 221 | [ADEGQSV] |
| 222 | [W] |
| 223 | [E] |
| 224 | [A] |
| 225 | [AEHKMQRST] |
| 226 | [L] |
| 227 | [RST] |
| 228 | [GNQS] |
| 229 | [ILMSTV] |
| 230 | [ST] |
| 231 | [EPST] |
| 232 | [ADEQ] |
| 233 | [AEGQRV] |
| 234 | [V] |
| 235 | [FIKLQRV] |
| 236 | [ADENQ] |
| 237 | [ADELMS] |
| 238 | [MWY] |
| 239 | [ILV] |
| 240 | [ADGKQRS] |
| 241 | [ADQ] |
| 242 | [T] |
| 243 | [P] |
| 244 | [L] |
| 245 | [AGR] |
| 246 | [R] |
| 247 | [IL] |
| 248 | [EQ] |
| 249 | [AELQRT] |
| 250 | [AP] |
| 251 | [DE] |
| 252 | [D] |
| 253 | [IV] |
| 254 | [A] |
| 255 | [DGKNRT] |
| 256 | [ALSV] |
| 257 | [AIV] |
| 258 | [AFISV] |
| 259 | [F] |
| 260 | [L] |
| 261 | [ACLV] |
| 262 | [GS] |
| 263 | [DEGNPS] |
| 264 | [ADLQS] |
| 265 | [AS] |
| 266 | [DGNR] |
| 267 | [F] |
| 268 | [IM] |
| 269 | [T] |
| 270 | [G] |
| 271 | [EQ] |
| 272 | [AG] |
| 273 | [ILV] |
| 274 | [AENS] |
| 275 | [V] |
| 276 | [NT] |
| 277 | [G] |
| 278 | [G] |
| 279 | [AV] |
| 280 | [FWY] |
| 281 | [IMT] |
| 282 | [DFT] |

The number of amino acid modifications in the polypeptide sequence is defined in the claims and can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 3, 34, 35, 36, 37, 38, 39, or 40 amino acids, while retaining or improving the catalytic activity of the polypeptide.

Polynucleotides which hybridize under stringent conditions to the full length complement of for example polynucleotides with nucleotide sequence SEQ ID NO:2 comprise polynucleotides which are detectable through diverse hybridization techniques, such as colony hybridization, plaque hybridization, Southern hybridization by using the complement full length DNA of SEQ ID NO: 2 as hybridization probe. A suitable hybridization method is described in Molecular Cloning, A laboratory manual, Second Edition (Cold Spring Harbor Laboratory Press, 1989).

For hybridization, the polynucleotide under investigation is immobilized on a filter and hybridized to the polynucleotide complement of e.g. SEQ ID No:2 in a 0.7-1 M NaCl solution at 60° C. Subsequently, the filter is washed with a 0.1 to 2-fold SSC solution at 65° C, wherein a 1-fold SSC solution is understood to be a mixture consisting of 150 mM NaCl and 15 mM sodium citrate.

A polynucleotide which hybridizes, for example, to the complement of SEQ ID No: 2 under stringent conditions has a nucleotide sequence, wherein at least 65 %, preferably at least 70% of nucleotides are identical to the nucleotides of SEQ ID NO:2.

A preferred embodiment of the invention is characterized in that the cofactor used in the process is continuously regenerated by the action of an oxidoreductase in the presence of secondary alcohol. Preferably, NADH is used as the cofactor, with the NAD formed in the reduction being reduced back to NADH.

The cofactor preference of an oxidoreductase for NADH could be changed to the preference for the NADPH and reverse by using mutagenesis techniques by mutating the cofactor binding site.

In the processes according to the invention, the oxidized cofactor NAD or NADP formed by the oxidoreductase/dehydrogenase is preferably regenerated continuously.

According to a preferred embodiment of the invention, the oxidized cofactor NAD or NADP is regenerated by oxidation of an alcohol.

Preferably, a secondary alcohol of general formula RₓR_{y}CHOH is used for cofactor regeneration, wherein Rₓ and R_{y} are independently selected from hydrogen, a branched or unbranched C₁-C₈-alkyl group, wherein the total number of carbon atoms Cₜₒₜₐₗ>=3.

Secondary alcohols such as 2-propanol, 2-butanol, 2-pentanol, 3-pentanol, 4-methyl-2-pentanol, 2-heptanol, 2-octanol or cyclohexanol are preferably used as cosubstrates. According to a particularly preferred embodiment, 2-propanol or 4-methyl-2-pentanol is used for coenzyme regeneration. The amount of cosubstrate for the regeneration can range in case of water miscible secondary alcohols from 5 to 70% by volume, preferably from 5 to 50 %, more preferably from 5 to 40 %, most preferably from 5 to 20 %, whereas in the case of water immiscible secondary alcohols such as Methyl-2-pentanol the preferred concentration ranges from 5 to 80 %, more preferred from 20 to 80 %, most preferred from 40 to 80% based on the total volume of the reaction batches.

According to a further preferred embodiment of the processes according to the invention, an additional oxidoreductase/dehydrogenase is added for the regeneration of the cofactor.

In a further preferred embodiment, a further alcohol dehydrogenase can, in addition, be added for the regeneration of the cofactor. Suitable NADH-dependent alcohol dehydrogenases are obtainable, for example, from baker's yeast, from Candida parapsilosis (CPCR) (U.S. Pat. No. 5,523,223 and U.S. Pat. No. 5,763,236, Enzyme Microb. Technol., 1993, 15(11):950-8), Pichia capsulata (EP1633779B1), from Rhodococcus erythropolis (RECR) (U.S. Pat. No. 5,523,223), Norcardia fusca (Biosci. Biotechnol. Biochem., 63(10), 1999, p. 1721-1729; Appl. Microbiol. Biotechnol., 2003, 62(4):380-6; Epub 2003, Apr. 26) or from Rhodococcus ruber (J. Org. Chem., 2003, 68(2):402-6). Suitable cosubstrates for those alcohol dehydrogenases are, for example, the already mentioned secondary alcohols such as 2-propanol (isopropanol), 2-butanol, 2-pentanol, 4-methyl-2-pentanol, 2-octanol or cyclohexanol.

Suitable secondary alcohol dehydrogenases for the regeneration of NADPH are, for example, those as described above and isolated from organisms of the order of Lactobacillales, e.g., Lactobacillus kefir (U.S. Pat. No. 5,200,335), Lactobacillus brevis (DE 19610984 A1; Acta Crystallogr. D. Biol. Crystallogr. 2000 December; 56 Pt 12:1696-8), Lactobacillus minor (DE 10119274), Leuconostoc carnosum (A 1261/2005, Kl. C12N) or, as described, those from Thermoanerobium brockii, Thermoanerobium ethanolicus or Clostridium beijerinckii.

However, other enzymatic systems can, in principle, also be used for cofactor regeneration. For example, cofactor regeneration can be effected using NAD- or NADP-dependent formate dehydrogenase (Tishkov et al., J. Biotechnol. Bioeng. [1999] 64, 187-193, Pilot-scale production and isolation of recombinant NAD and NADP specific formate dehydrogenase). Suitable cosubstrates of formate dehydrogenase are, for example, salts of formic acid such as ammonium formate, sodium formate or calcium formate.

For the conversion of 1 kg of the Substrate Quinuclidin-3-one 5000 to 10 Mio.Units of Oxidoreductase should be applied. The enzyme activity unit (U) is defined as amount of enzyme required for the conversion of 1 µmol of Quinuclidin-3-one to the corresponding alcohol pro minute.

In the processes according to the invention, the substrate Quinuclidin-3-one is used in the reaction batch preferably in an amount of from 10 g/l to 500 g/l, preferably from 25 g/l to 300 g/l, particularly preferably from 50 g/l to 200 g/l, based on the total volume.

The aqueous portion of the reaction mixture in which the enzymatic reduction proceeds preferably contains a buffer, e.g., a potassium phosphate, tris/HCl or triethanolamine buffer, having a pH value of from 5 to 10, preferably a pH of from 6 to 9. In addition, the buffer can contain ions for stabilizing or activating the enzymes such as, for example, zinc ions or magnesium ions.

While carrying out the process according to the invention, the temperature suitably ranges from about 10° C. to 70° C., preferably from 20° C. to 45° C.

The concentration of the cofactor, in particular of NADH or NADPH, respectively, in the aqueous phase generally ranges from 0.001 mM to 10 mM.

The TTN (total turnover number=mol of reduced compound of formula l/mol of cofactor used) achieved in the processes according to the invention normally ranges from 10² to 10⁵, preferably, however, it is >=10³.

In the process according to the invention, a stabilizer of oxidoreductase/dehydrogenase can also be used. Suitable stabilizers are, for example, glycerol, sorbitol, 1,4-DL-dithiothreitol (DTT) or dimethyl sulfoxide (DMSO).

According to another possible embodiment of the invention, the oxidized cosubstrate (e.g. acetone) can be removed continuously and/or the cosubstrate (e.g. 2-propanol) can be newly added in a continuous manner in order to shift the reaction equilibrium towards the reaction product.

After completion of the reduction, the reaction mixture is processed. For this purpose, e.g., the aqueous phase is optionally separated from the organic phase and the organic phase containing the product is filtered. Optionally, the aqueous phase can also be extracted and processed further like the organic phase. Thereupon, the solvent is evaporated from the organic phase and the product of general formula II or III is obtained as a crude product. The crude product can then be purified further or used directly for the synthesis of a resultant product.

In the following, the invention is illustrated further by way of examples.

### EXAMPLE 1

### Isolation of Oxidoreductase from Mycobacterium vanbaalenii

In order to isolate the NADH-dependent oxidoreductase from Mycobacterium vanbaalenii the microorganism was cultivated in Peptone 5.0 g, Meat extract 3.0 g pro Liter Water, pH7.0.

Upon reaching the stationary phase, the cells were harvested and separated from the medium by centrifugation. 5 g of Mycobacterium vanbaalenii cells were suspended with 20 ml of buffer (100 mM triethanolamine, 1 mM MgCl2, pH=7.0) and homogenized. The crude extract obtained after centrifugation (7000 rpm) was then purified further and reprocessed via FPLC (fast protein liquid chromatography). The oxidoreductase could be purified in four consecutive steps via ion exchange chromatography on Butyl-Sepharose (Messrs. Pharmacia), two times purification on Q-Sepharose Fast Flow (Messrs. Pharmacia) with following Hydroxyapatite-Chromatogrophy (Bio-Rad) . For this purpose, the lysate obtained after the centrifugation was directly applied to a Butyl-Sepharose column equilibrated with 50 mM of a TEA, 1 mM MgCl₂, 1M Ammoniumsulfat pH=7.0, and was eluted with an decreasing linear salt gradient. In doing so, the oxidoreductase was eluted at 0.2 to 0.3 M Ammoniumsulfat. The oxidoreductase-containing fractions were combined and concentrated to an appropriate volume by means of ultrafiltration (exclusion limit 10 kDa). Subsequently, the fractions of oxidoreductase which had been concentrated were reprocessed and purified further via Q-Sepharose, using 50 mM Kaliumphosphate Buffer with 1 mM MgCl₂ and 1 M NaCl. The enzyme was thereby eluted at 0.7-0.8 M NaCl. Subsequently the active fractions were concentrated and equilibrated with 10 mM Kaliumphosphate Bufer, pH 7.0, 1 mM MgCl₂ and applied onto the Hydroxyapatite-Column and eluted with 200 mM Kaliumphosphate Buffer, 1 mM MgCl₂. The following Table summarizes the results obtained.

**TABLE**

| Purification Step | Protein concentration | Total Protein (mg) | Total Activity | Specific activity |
|---|---|---|---|---|
| Cell Lysate | 8.9 | 89 | 250 | 2.8 |
| Butyl-Sepha rose | 5 | 7.5 | 115 | 15 |
| Q-Sepharose | 1.4 | 1.68 | 58 | 34 |
| Hydroxyapatite | 0.5 | 0.25 | 23 | 92 |

The determination of the protein amount was performed according to Lowry et al. Journal of Biological Chemistry, 193 (1951): 265-275 or Peterson et al., Analytical Biochemistry, 100 (1979): 201-220). The quotient of enzyme activity to protein amount yields the specific activity, wherein the conversion of 1 µmol per min corresponds to 1 unit (U).

### EXAMPLE 2

### Determination of the N-Terminal Sequence of the Oxidoreductase According to the Invention

After Hydroxyapaptite-Column purification step, the enzyme preparation according to Example 1 was fractioned in a 10% sodium dodecyl sulfate (SDS) gel and transferred onto a polyvinylidene difluoride membrane (PVDF-membrane).

The conspicuous band at about 30 kDa was subjected to N-terminal sequencing via Edman degradation (Procise 492 (PE-Biosystems)) and the sequencing of prominent internal peptide. The following N-terminal sequence was obtained:

### TRTVVVTGAGSGIGR

Sequence of internal peptide:

### LEQADDVAR

### EXAMPLE 3

### a) Cloning of the Oxidoreductase from Mycobacterium vanbaalenii

Genomic DNA isolated from the cells of Mycobacterium vanbaalenii PYR-1 was used as a template for PCR reaction with degenerated primers designed based on the protein fragment sequences from the edman- degradation. In doing so, amplification was carried out in a PCR buffer [16 mM (NH4)2SO4; 67 mM Tris-HCl pH 8.3 (at 25[deg.] C.); 1.5 m MgCl2; 0.01% Tween 20; 0.2 mM dNTP Mix; in each case 30 pMol of primer and 1.25 U of BioTherm Star Polymerase (Genecraft)] with 50 ng of genomic DNA as template.
Cycle 1: 95° C, 7 min
Cycle 2 x 28: 94° C, 40 sec
Temperature drop start 63° C -0.5° C/step, 30 sec
68° C., 60 sec
Cycle 3 x 20: 94° C, 40 sec
53° C, 40 sec
72° C, 60 sec
Cycle 4: 70° C, 7 min
4° C, [infinity]

After the fractionation of the entire PCR batch in the 1% agarose gel, a band of about 650 by was identified and cloned via overhanging adenosine moieties into a Topo-TA vector (Invitrogen) for the determination of the DNA sequence.

The DNA band resulting from the screening reaction exhibited an open reading frame corresponding to the fragment of an oxidoreductase of 227 amino acid residues.

The determination of the entire sequence coding for the oxidoreductase was accomplished by Inverted PCR (iPCR) method.

The gene sequence coding for the oxidoreductase included 771 base pairs and was equivalent to a length of 256 amino acid residues.

### b) Synthesis of a Full-Length DNA Fragment Coding for a Short-Chain ADH from Mycobacterium vanbaalenii by PCR

Specific primers were constructed for subsequent cloning of the full-length transcript into the appropriate expression systems. In doing so, a 5'-primer with a recognition sequence for Nde I and a 3'-primer with a recognition sequence for Hind III were modified. Genomic DNA isolated from the cells of Mycobacterium vanbaalenii served as a template for the polymerase chain reaction. Amplification was carried out in a PCR buffer [10 mM Tris-HCl (pH 8.0); 50 mM KCI; 10 mM MgSO4; 1 mM dNTP Mix; in each case 20 pMol of primer and 2.5 U of Platinum Pfx DNA Polymerase (Invitrogen)] with 50 ng of template and the following temperature cycles:
Cycle 1: 94° C, 2 min
Cycle 2 x 30: 94° C, 15 sec
58° C, 30 sec
68° C, 75 sec
Cycle 3: 68° C, 7 min
4° C, [infinity]

The resulting PCR product was restricted after purification over a 1% agarose gel with the aid of the endonucleases Nde I and Hind III and was ligated into the backbone of the pET21a vector (Novagen), which backbone had been treated with the same endonucleases. After transforming 2 [mu]I of the ligation batch into E. coli Top 10 F' cells (Invitrogen), plasmid DNAs of ampicillin- (or kanamycin) resistant colonies were tested for the presence of an insert having a size of 750 by means of a restriction analysis with the endonucleases Nde I and Hind. The expression constructs pET21-Myc was sequenced. The gene from mycobacterium vanbalenii coding for a short-chain oxidoreductase had an open reading frame of a total of 771 by (SEQ ID No: 2), which corresponded to a protein of 256 amino acids (SEQ ID No: 1).

### EXAMPLE 4.

### Expression of Recombinant Oxidoreductase in E. Coli Cells

Competent Escherichia coli Star BL21 (De3) cells (Invitrogen) were transformed with the expression constructs pET21-MIX coding for the oxidoreductase. The Escherichia coli cells transformed with the expression constructs were then cultivated in 200 ml of LB medium (1% tryptone, 0.5% yeast extract, 1% NaCl) with 50µg/ml of ampicillin or 40 µg/ml of kanamycin, respectively, until an optical density of 0.5, measured at 550 nm, was reached. The expression of recombinant protein was induced by adding isopropylthiogalactoside (IPTG) with a concentration of 0.1 mM. After 16 hours of induction at 25° C. and 220 rpm, the cells were harvested and frozen at -20° C. For the activity test, 10 mg of cells were mixed with 500 µl of 100 mM TEA buffer pH 7.0, 1 mM MgCl₂ and 500 µl glass beads and disrupted for 10 min using a glass mill. The lysate obtained was then used in a diluted state for the respective measurements.

The activity test was made up as follows: 870 µl of 100 mM TEA buffer pH 7.0, 1 mM MgCl2, 160 µg NADH, 10 µl of diluted cell lysate. The reaction was started by adding 100 µl of a 100 mM substrate solution to the reaction mixture.

For enzyme recovery in large amounts, 30 g of cells were resuspended in 150 ml of triethanolamine (TEA) buffer (100 mM, pH 7, 2 mM MgCl2, 10% glycerol) and disrupted using a high-pressure homogenizer. Subsequently, the enzyme solution was mixed with 150 ml glycerol and stored at -20° C.

### EXAMPLE 5

### Determination of oxidoreductase activity for reduction of Quinuclidin-3-one by photometric assay.

The activity of enzymes prepared as described in Example 4 was determined as activity of oxidation of 1 µmol of NADH to NAD in one minute by adding the substrate 3-Quinuclidinone (10 mM), a coenzyme NADH (0.25 mM) to the 10 µl of enzyme solution in 1 ml 100 mM TEA Buffer, pH 7.0 supplemented with 1 mM MgCl₂. The rate of absorbance decrease at 340 nm per minute divided through absorbance coefficient for NADH (6.22 M⁻¹cm⁻¹) is proportional to the enzyme activity (U) for reduction of Quinuclidin-3-one to corresponding alcohol.

| **Oxidoreductase:** | **Activity (U/g wet weight)** |
|---|---|
| **SEQ ID NO: 1** | **9000 U/g** |
| **SEQ ID NO: 3** | **281 U / g** |
| **SEQ ID NO: 5** | **1088 U / g** |
| **SEQ ID NO: 7** | **413 U / g** |
| **SEQ ID NO: 9** | **5000 U/g** |

### EXAMPLE 6

### Characterization of Oxidoreductases with regard to the Reduction Properties of the Quinuclidin-3-one

The oxidoreductases of polypeptide sequences SEQ ID No: 1, SEQ ID NO: 3, SEQ ID NO: 5, SEQ ID NO: 7 and SEQ ID NO: 9 were examined for the determination of the enantioselectivity and conversion value by the reduction of Quinuclidin-3-one to the corresponding alcohol by following procedure:
Recombinant Escherichia coli carrying the genes of oxidoreductases of SEQ ID NO: 1, SEQ ID NO: 3, SEQ ID NO: 5, SEQ ID NO: 7 and SEQ ID NO: 9 were cultured as described in example 4. 18 h postinduction 2 g of each recombinant strains cells were resuspended in 10ml 100 mM triethanolamine Buffer, pH 7.0 supplemented with 2 mM MgCl₂ and disrupted using the glass mill for 10 min. Resulted enzyme solution was clarified by centrifugation and mixed with equal volume of glycerol. For the reaction setup 56 µl of enzyme solution was used for the conversion of 75 mg Quinuclidin-3-one by adding 0.15 mg NAD, 75 µl (10 %) Oxidoreductase aus Pichia capsulata, 250 µl Methyl-2-pentanol and 119 µl 100 mM TEA pH 6.5 supplemented with 2 mM MgCl₂. After 24 h of incubating of samples at 30 ° C 10 µl of the reaction was added to 1 ml MeOH and GC analyzed. For the detection of the enantiomers Hydrodex β-6TBDM column (25 m x 0.25 mm x 0.25 µm) from Masherey-Nagel (Germany) was used.

| SEQ No: | SEQ ID NO:1 | SEQ ID NO: 3 | SEQ ID NO:5 | SEQ ID NO:7 | SEQ ID NO: 9 |
|---|---|---|---|---|---|
| Ee (%) | > 99 | > 95 | > 95 | > 95 | > 95 |
| Conversion (%) | 100 | > 78 | > 81 | 66 | > 84 |

### EXAMPLE 7

### Conversion of Quinuclidin-3-one to the corresponding (R)-3-Quinuclidinol in 10 ml scale with Methyl-2-pentanol as cosubstrate

Recombinant Escherichia coli carrying the gene of oxidoreductase of SEQ ID NO: 1 was cultured as described in example 4. 18 hours postinduction 30 g of harvested cells were resuspendet in 100 ml of 100 mM triethanolamine (TEA) Buffer, pH 7.0 supplemented with 2 mM MgCl₂ and disrupted with French press. The obtained lysate was clarified by centrifugation at 6000 g for 10 min at 4 ° C. For the reduction of 1.5 g 3-Quinuclidinone 250 µl of enzyme solution (1125 U) was added to the 4.05 ml 100 mM TEA buffer, pH 6.5 supplemented with 2 mM MgCl₂, 3 mg NAD, 225 U of Alcohol Dehydrogenase from Pichia capsulata and 5 ml Methyl-2-pentanol. The reaction was incubated under stirring by 30 ° C. After 21 hours the reaction was stopped through addition of 3 ml 5 M NaOH, 5 ml of Methanol and analyzed by gas chromatography. The measured rate of conversion to (R)-3-Quinuclidinol was 98 % with the optical purity of the product of > 99 %.

### EXAMPLE 8

### Conversion of Quinuclidin-3-one to the corresponding (R)-3-Quinuclidinol in 10 ml scale with Isopropanol as cosubstrate

Recombinant Escherichia coli carrying the gene of oxidoreductase of SEQ ID NO: 1 was cultured as described in example 4. 18 hours postinduction 30 g of harvested cells were resuspendet in 100 ml of 100 mM triethanolamine (TEA) Buffer, pH 7.0 supplemented with 2 mM MgCl₂ and disrupted with French press. For the reduction of 1.5 g Quinuclidin-3-one 525 U of enzyme was added to the 5.25 ml 100 mM TEA buffer, pH 7.0 supplemented with 1 mM ZnCl₂, 1.5 mg NAD, 525 U of Alcohol Dehydrogenase from Candida nemodendra (WO2007012428, SEQ ID NO: 8) and 1 ml Isopropanol. The reaction was incubated under stirring by 30 ° C. After 24 h of incubation the reaction was stopped through addition of 3 ml 5 M NaOH, 5 ml of Methanol and analyzed by gas chromatography. The measured rate of conversion to (R)-3-Quinuclidinol was 100 % with the optical purity of the product of > 99 %.

## Claims

1. Process for production of (R)-3-Quinuclidinol or a salt thereof by reduction of Quinuclidin-3-one with cofactor and oxidoreductase, **characterized in that**, the oxidoreductase comprises an amino acid sequence motif MQX₁REX₂X₃WEA, wherein each of X₁, X₂, X₃ are any of amino acid residues A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) or V (Val), and **in that** the oxidoreductase is selected from
a) polypeptides with an amino acid sequence, wherein at least 65 % of the amino acids are identical to the amino acids of SEQ ID NO: 1 or SEQ ID NO:3; or
b) polypeptides with an amino acid sequence, wherein at least 75 % of the amino acids are identical to the amino acids of SEQ ID NO:5 or SEQ ID NO:9; or
c) polypeptides with an amino acid sequence, wherein at least 85 % of amino acids are identical to the amino acids of SEQ ID NO:7.

2. Process for production of (R)-3-Quinuclidinol or a salt thereof by reduction of Quinuclidin-3-one with cofactor and oxidoreductase, **characterized in that**, the oxidoreductase comprises an amino acid sequence motif MQX₁REX₂X₃WEA, wherein each of X₁, X₂, X₃ are any of amino acid residues A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) or V (Val), and **in that** the oxidoreductase is selected from
a) polypeptides with an amino acid sequence derived from SEQ ID NO: 1 or SEQ ID NO:3 or SEQ ID NO: 5 or SEQ ID NO:7 or SEQ ID NO:9 through substitution, deletion or addition of one, two, three, four, five, six, seven, eight, nine, ten, eleven, twelve, thirteen, fourteen, fifteen, sixteen, seventeen, eighteen, nineteen, twenty, twentyone, twentytwo, twentythree, twentyfour, twentyfive, twentysix or twentyseven to forty amino acid residues, which are capable of reducing Quinuclidin-3-one; or
b) polypeptides which are encoded by a polynucleotide with nucleotide sequence SEQ ID NO: 2 or SEQ ID NO:4 or SEQ ID NO:6 or SEQ ID NO:8 or SEQ ID NO:10 of the sequence listing; or
c) polypeptides which are encoded by a polynucleotide with a nucleotide sequence that hybridizes to the full length complement of nucleotide sequence SEQ ID NO: 2 or SEQ ID NO:4 or SEQ ID NO:6 or SEQ ID NO:8 or SEQ ID NO:10 under stringent conditions, wherein stringent conditions comprise hybridization in 0.7-1 M NaCl solution at 60° C and washing in a 0.1 to 2-fold SSC solution at 65° C, and a 1-fold SSC solution is understood to be a mixture consisting of 150 mM NaCl and 15 mM sodium citrate.

3. Process for production of (R)-3-Quinuclidinol or a salt thereof by reduction of Quinuclidin-3-one with cofactor and oxidoreductase, **characterized in that**, the oxidoreductase comprises an amino acid sequence motif MQX₁REX₂X₃WEA, wherein each of X₁, X₂, X₃ are any of amino acid residues A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) or V (Val), and **in that** the oxidoreductase is selected from
polypeptides with amino acid sequence SEQ ID NO:1 or SEQ ID NO:3 or SEQ ID NO:5 or SEQ ID NO:7 or SEQ ID NO: 9 of the sequence listing.

4. A process according to any of claims 1 to 3, **characterized in that** the cofactor is continuously regenerated with a cosubstrate.

5. A process according to any of claims 1 to 4, **characterized in that** NADH or NADPH is used as cofactor.

6. A process according to any of claims 1 to 5, **characterized in that** 2-propanol, 2-butanol, 2-pentanol, 4-methyl-2-pentanol, 2-heptanol or 2-octanol is used as a cosubstrate or as a secondary alcohol, respectively.

7. A process according to any of claims 1 to 6, **characterized in that** the secondary alcohol as cosubstrate is used in an amount from 5 to 70% by volume based on the total volume of the reaction batches.

8. A process according to claim 7, **characterized in that** the secondary alcohol as cosubstrate is used in an amount from 5 to 50% by volume based on the total volume of the reaction batches.

9. A process according to any of claims 1 to 6, **characterized in that** the secondary alcohol used as cosubstrate is water immiscible and is used in an amount from 5 to 80% by volume based on the total volume of the reaction batches.

10. A process according to claim 9, **characterized in that** the secondary alcohol used as cosubstrate is water immiscible and is used in an amount from 20 to 80% by volume based on the total volume of the reaction batches.

11. A process according to claim 9 or 10, **characterized in that** the secondary alcohol used as cosubstrate is Methyl-2-pentanol.

12. A process according to any of claims 1 to 11, **characterized in that** the Quinuclidin-3-one is used in an amount of from 5 to 50% by weight, based on the total reaction volume.

13. A process according to claim 12, **characterized in that** the Quinuclidin-3-one is used in an amount of from 8-40% by weight based on the total reaction volume.

14. A process according to any of claims 1 to 13, **characterized in that** the total turnover number TTN (= mol of reduced Quinuclidin-3-one per mol of cofactor) is >10³.

15. A process according to any of claims 1 to 14, **characterized in that** it is carried out in an aqueous organic two-phase system.

16. A process according to any of claims 1 to 15, **characterized in that**, in addition, an organic solvent is used.

17. A process according to claim 16, **characterized in that**, diethyl ether, tertiary butyl methyl ether, diisopropyl ether, dibutyl ether, ethyl acetate, butyl acetate, heptane, hexane or cyclohexane is used as organic solvent.

18. A process according to any of claims 1 to 17, **characterized in that** a further oxidoreductase is added in the reaction for cofactor regeneration.

## Patentansprüche

1. Verfahren zum Herstellen von (R)-3-Chinuclidinol oder einem Salz davon durch Reduktion von Chinuclidin-3-on mit Cofaktor und Oxidoreduktase, **dadurch gekennzeichnet, dass** die Oxidoreduktase ein Aminosäure-Sequenzmotiv MQX₁REX₂X₃WEA umfasst, wobei jedes von X₁, X₂, X₃ ein beliebiger der Aminosäurereste A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) oder V (Val) ist und dass die Oxidoreduktase ausgewählt ist aus
a) Polypeptiden mit einer Aminosäuresequenz, bei der wenigstens 65 % der Aminosäuren mit den Aminosäuren von SEQ ID NO: 1 oder SEQ ID NO: 3 identisch sind; oder
b) Polypeptiden mit einer Aminosäuresequenz, bei der wenigstens 75 % der Aminosäuren mit den Aminosäuren von SEQ ID NO:5 oder SEQ ID NO:9 identisch sind; oder
c) Polypeptiden mit einer Aminosäuresequenz, bei der wenigstens 85 % der Aminosäuren mit den Aminosäuren von SEQ ID NO:7 identisch sind.

2. Verfahren zum Herstellen von (R)-3-Chinuclidinol oder einem Salz daron durch Reduktion von Chinuclidin-3-on mit Cofaktor und Oxidoreduktase, **dadurch gekennzeichnet, dass** die Oxidoreduktase ein Aminosäure-Sequenzmotiv MQX₁REX₂X₃WEA umfasst, wobei jedes von X₁, X₂, X₃ ein beliebiger der Aminosäurereste A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) oder V (Val) ist und dass die Oxidoreduktase ausgewählt ist aus
a) Polypeptiden mit einer Aminosäuresequenz abgeleitet von SEQ ID NO:1 oder SEQ ID NO:3 oder SEQ ID NO:5 oder SEQ ID NO:7 oder SEQ ID NO:9 durch Substitution, Deletion oder Addition von einem, zwei, drei, vier, fünf, sechs, sieben, acht, neun, zehn, elf, zwölf, dreizehn, vierzehn, fünfzehn, sechzehn, siebzehn, achtzehn, neunzehn, zwanzig, einundzwanzig, zweiundzwanzig, dreiundzwanzig, vierundzwanzig, fünfundzwanzig, sechsundzwanzig oder siebenundzwanzig bis vierzig Aminosäureresten, die zum Reduzieren von Chinuclidin-3-on fähig sind; oder
b) Polypeptiden, die von einem Polynukleotid mit der Nukleotidsequenz SEQ ID NO:2 oder SEQ ID NO:4 oder SEQ ID NO:6 oder SEQ ID NO:8 oder SEQ ID NO:10 der Sequenzliste kodiert werden; oder
c) Polypeptiden, die von einem Polynukleotid mit einer Nukleotidsequenz kodiert werden, die mit dem Komplement voller Länge der Nukleotidsequenz SEQ ID NO:2 oder SEQ ID NO:4 oder SEQ ID NO:6 oder SEQ ID NO:8 oder SEQ ID NO:10 unter stringenten Bedingungen hybridisiert, wobei stringente Bedingungen Hybridisieren in 0,7-1 M NaCl-Lösung bei 60 °C und Waschen in einer 0,1- bis 2-fachen SSC-Lösung bei 65 °C umfasst und eine 1-fache SSC-Lösung ein Gemisch bedeuten soll, das aus 150 mM NaCl und 15 mM Natriumcitrat besteht.

3. Verfahren zum Herstellen von (R)-3-Chinuclidinol oder einem Salz daron durch Reduktion von Chinuclidin-3-on mit Cofaktor und Oxidoreduktase, **dadurch gekennzeichnet, dass** die Oxidoreduktase ein Aminosäure-Sequenzmotiv MQX₁REX₂X₃WEA umfasst, wobei jedes von X₁, X₂, X₃ ein beliebiger der Aminosäurereste A (Ala), R (Arg), N (Asn), D (Asp), C (Cys), Q (Gln), E (Glu), G (Gly), H (His), I (Ile), L (Leu), K (Lys), M (Met), F (Phe), P (Pro), S (Ser), T (Thr), W (Trp), Y (Tyr) oder V (Val) ist und dass die Oxidoreduktase ausgewählt ist aus Polypeptiden mit der Aminosäuresequenz SEQ ID NO:1 oder SEQ ID NO:3 oder SEQ ID NO:5 oder SEQ ID NO:7 oder SEQ ID NO:9 der Sequenzliste.

4. Verfahren gemäß einem der Ansprüche 1 bis 3, **dadurch gekennzeichnet, dass** der Cofaktor kontinuierlich mit einem Cosubstrat regeneriert wird.

5. Verfahren gemäß einem der Ansprüche 1 bis 4, **dadurch gekennzeichnet, dass** NADH oder NADPH als Cofaktor verwendet wird.

6. Verfahren gemäß einem der Ansprüche 1 bis 5, **dadurch gekennzeichnet, dass** 2-Propanol, 2-Butanol, 2-Pentanol, 4-Methyl-2-pentanol, 2-Heptanol oder 2-Octanol als Cosubstrat bzw. sekundärer Alkohol verwendet wird.

7. Verfahren gemäß einem der Ansprüche 1 bis 6, **dadurch gekennzeichnet, dass** der sekundäre Alkohol als Cosubstrat in einer Menge von 5 bis 70 Vol.-%, bezogen auf das Gesamtvolumen der Reaktionschargen, verwendet wird.

8. Verfahren gemäß Anspruch 7, **dadurch gekennzeichnet, dass** der sekundäre Alkohol als Cosubstrat in einer Menge von 5 bis 50 Vol-%, bezogen auf das Gesamtvolumen der Reaktionschargen, verwendet wird.

9. Verfahren gemäß einem der Ansprüche 1 bis 6, **dadurch gekennzeichnet, dass** der sekundäre Alkohol, der als Cosubstrat verwendet wird, nicht mit Wasser mischbar ist und in einer Menge von 5 bis 80 Vol.-%, bezogen auf das Gesamtvolumen der Reaktionschargen, verwendet wird.

10. Verfahren gemäß Anspruch 9, **dadurch gekennzeichnet, dass** der sekundäre Alkohol, der als Cosubstrat verwendet wird, nicht mit Wasser mischbar ist und in einer Menge von 20 bis 80 Vol-%, bezogen auf das Gesamtvolumen der Reaktionschargen, verwendet wird.

11. Verfahren gemäß Anspruch 9 oder 10, **dadurch gekennzeichnet, dass** der sekundäre Alkohol, der als Cosubstrat verwendet wird, Methyl-2-pentanol ist.

12. Verfahren gemäß einem der Ansprüche 1 bis 11, **dadurch gekennzeichnet, dass** das Chinuclidin-3-on in einer Menge von 5 bis 50 Gew.-%, bezogen auf das gesamte Reaktionsvolumen, verwendet wird.

13. Verfahren gemäß Anspruch 12, **dadurch gekennzeichnet, dass** das Chinuclidin-3-on in einer Menge von 8 bis 40 Gew.-%, bezogen auf das gesamte Reaktionsvolumen, verwendet wird.

14. Verfahren gemäß einem der Ansprüche 1 bis 13, **dadurch gekennzeichnet, dass** die Gesamtwechselzahl TTN (= mol an reduziertem Chinuclidin-3-on pro mol Cofaktor) >10³ beträgt.

15. Verfahren gemäß einem der Ansprüche 1 bis 14, **dadurch gekennzeichnet, dass** es in einem wässrigorganischen Zweiphasensystem durchgeführt wird.

16. Verfahren gemäß einem der Ansprüche 1 bis 15, **dadurch gekennzeichnet, dass** zusätzlich ein organisches Lösungsmittel verwendet wird.

17. Verfahren gemäß Anspruch 16, **dadurch gekennzeichnet, dass** Diethylether, tertiärer Butylmethylether, Diisopropylether, Dibutylether, Ethylacetat, Butylacetat, Heptan, Hexan oder Cyclohexan als organisches Lösungsmittel verwendet wird.

18. Verfahren gemäß einem der Ansprüche 1 bis 17, **dadurch gekennzeichnet, dass** zu der Reaktion eine weitere Oxidoreduktase zur Cofaktor-Regeneration zugegeben wird.

## Revendications

1. Procédé de production de (R)-3-quinuclidinol ou d'un sel de celui-ci par réduction de quinuclidin-3-one avec un cofacteur et une oxydoréductase, **caractérisé en ce que** l'oxydoréductase comprend un motif de séquence d'acides aminés MQX₁REX₂X₃WEA, dans lequel chacun de X₁, X₂, X₃ est n'importe lequel des résidus d'acides aminés A (ALA), R (ARG), N (ASN), D (ASP), C (CYS), Q (GIN), E (GLU), G (GLY), H (HIS), I (ILE), L (LEU), K (LYS), M (MET), F (PHE), P (PRO), S (SER), T (THR), W (TRP), Y (TYR) or V (VAL), et **en ce que** l'oxydoréductase est choisie parmi
A) des polypeptides avec une séquence d'acides aminés, dans lequel au moins 65 % des acides aminés sont identiques aux acides aminés de SEQ ID No.: 1 ou SEQ ID No.: 3 ; ou
B) des polypeptides avec une séquence d'acides aminés, dans lequel au moins 75 % des acides aminés sont identiques aux acides aminés de SEQ ID No. : 5 ou de SEQ ID No. : 9 ; ou
C) des polypeptides avec une séquence d'acides aminés, dans lequel au moins 85 % des acides aminés sont identiques aux acides aminés de SEQ ID No.: 7.

2. Procédé de production de (R)-3-quinuclidinol ou d'un sel de celui-ci par réduction de quinuclidin-3-one avec un cofacteur et une oxydoréductase, **caractérisé en ce que** l'oxydoréductase comprend un motif de séquence d'acides aminés MQX₁REX₂X₃WEA, dans lequel chacun de X₁, X₂, X₃ est n'importe lequel des résidus d'acides aminés A (ALA), R (ARG), N (ASN), D (ASP), C (CYS), Q (GIN), E (GLU), G (GLY), H (HIS), I (ILE), L (LEU), K (LYS), M (MET), F (PHE), P (PRO), S (SER), T (THR), W (TRP), Y (TYR) or V (VAL), et **en ce que** l'oxydoréductase est choisie parmi
A) des polypeptides avec une séquence d'acides aminés dérivée de SED ID No. : 1 ou SEQ ID No.: 3 ou SEQ ID No.: 5 ou SEQ ID No.: 7 ou SEQ ID No. : 9 par le biais d'une substitution, délétion ou addition d'un, deux, trois, quatre, cinq, six, sept, huit, neuf, dix, onze, douze, treize, quatorze, quinze, seize, dix-sept, dix-huit, dix-neuf, vingt, vingt-et-un, vingt-deux, vingt-trois, vingt-quatre, vingt-cinq ou vingt-six à quarante résidus d'acides aminés, qui sont capables de réduire la quinuclidin-3-one ; ou
B) des polypeptides qui sont codés par un polynucléotide avec la séquence de nucléotides SEQ ID No. : 2 ou SEQ ID No. : 4 ou SEQ ID No. : 6 ou SEQ ID No. : 8 ou SEQ ID No. : 10 du listage des séquences ; ou
C) des polypeptides qui sont codés par un polynucléotide avec une séquence de nucléotides qui s'hybride au complément de longueur complète de la séquence de nucléotides SEQ ID No. : 2 ou SEQ ID No. : 4 ou SEQ ID No. : 6 ou SEQ ID No. : 8 ou SEQ ID No. : 10 dans des conditions stringentes, dans lequel des conditions stringentes comprennent une hybridation dans une solution de nacl 0,7-1 M à 60 °C et lavage dans une solution de SSC à 0,1 à 2 fois à 65 °C, et une solution de SSC à 1 fois est comprise comme étant un mélange constitué de 150 mM de NACL et 15 mM de citrate de sodium.

3. Procédé de production de (R)-3-quinuclidinol ou d'un sel de celui-ci par réduction de quinuclidin-3-one avec un cofacteur et une oxydoréductase, **caractérisé en ce que** l'oxydoréductase comprend un motif de séquence d'acides aminés MQX₁REX₂X₃WEA, dans lequel chacun de X₁, X₂, X₃ est n'importe lequel des résidus d'acides aminés A (ALA), R (ARG), N (ASN), D (ASP), C (CYS), Q (GIN), E (GLU), G (GLY), H (HIS), I (ILE), L (LEU), K (LYS), M (MET), F (PHE), P (PRO), S (SER), T (THR), W (TRP), Y (TYR) or V (VAL), et **en ce que** l'oxydoréductase est choisie parmi
des polypeptides avec la séquence d'acides aminés SEQ ID No.: 1 ou SEQ ID No. : 3 ou SEQ ID No. : 5 ou SEQ ID No. : 7 ou SEQ ID No. : 9 du listage des séquences.

4. Procédé selon l'une quelconque des revendications 1 à 3, **caractérisé en ce que** le cofacteur est régénéré de façon continue avec un cosubstrat.

5. Procédé selon l'une quelconque des revendications 1 à 4, **caractérisé en ce que** nadh ou nadph est utilisé en tant que cofacteur.

6. Procédé selon l'une quelconque des revendications 1 à 5, **caractérisé en ce que** du 2-propanol, du 2-butanol, du 2-pentanol, du 4-méthyl-2-pentanol, du 2-heptanol ou du 2-octanol est utilisé en tant que cosubstrat ou en tant qu'alcool secondaire, respectivement.

7. Procédé selon l'une quelconque des revendications 1 à 6, **caractérisé en ce que** l'alcool secondaire en tant que cosubstrat est utilisé en une quantité allant de 5 à 70 % en volume sur la base du volume total des lots de réaction.

8. Procédé selon la revendication 7, **caractérisé en ce que** l'alcool secondaire en tant que cosubstrat est utilisé en une quantité allant de 5 à 50 % en volume sur la base du volume total des lots de réaction.

9. Procédé selon l'une quelconque des revendications 1 à 6, **caractérisé en ce que** l'alcool secondaire utilisé en tant que cosubstrat est non miscible dans l'eau et est utilisé en une quantité allant de 5 à 80 % en volume sur la base du volume total des lots de réaction.

10. Procédé selon la revendication 9, **caractérisé en ce que** l'alcool secondaire utilisé en tant que cosubstrat est non miscible dans l'eau et est utilisé en une quantité allant de 20 a 80 % en volume sur la base du volume total des lots de réaction.

11. Procédé selon la revendication 9 ou 10, **caractérisé en ce que** l'alcool secondaire utilisé en tant que cosubstrat est le méthyl-2-pentanol.

12. Procédé selon l'une quelconque des revendications 1 à 11, **caractérisé en ce que** la quinuclidin-3-one est utilisée en une quantité allant de 5 à 50 % en poids, sur la base du volume total de réaction.

13. Procédé selon la revendication 12, **caractérisé en ce que** la quinuclidin-3-one est utilisée en une quantité allant de 8 à 40 % en poids, sur la base du volume total de réaction.

14. Procédé selon l'une quelconque des revendications 1 à 13, **caractérisé en ce que** le nombre total d'échanges TTN (= mol de quinudidin-3-one réduite par mol de cofacteur) est >10³.

15. Procédé selon l'une quelconque des revendications 1 à 14, **caractérisé en ce qu'**il est effectué dans un système aqueux organique à deux phases.

16. Procédé selon l'une quelconque des revendications 1 à 15, **caractérisé en ce que** l'on utilise en outre un solvant organique.

17. Procédé selon la revendication 16, **caractérisé en ce que** de l'éther diéthylique, de l'éther butyl-méthylique tertiaire, de l'éther di-isopropylique, de l'éther dibutylique, de l'acétate d'éthyle, de l'acétate de butyle, de l'heptane, de l'hexane ou du cyclohexane est utilisé en tant que solvant organique.

18. Procédé selon l'une quelconque des revendications 1 à 17, **caractérisé en ce qu'**une oxydoréductase supplémentaire est ajoutée dans la réaction pour la régénération du cofacteur.
